# EUROPÄISCHE PATENTSCHRIFT

(11) **EP 3 576 875 B1**
(45) Veröffentlichungstag und Bekanntmachung des Hinweises auf die Patenterteilung: **04.06.2025**
(21) Anmeldenummer: 18725741.5
(22) Anmeldetag: 26.01.2018
(51) Int. Cl.: B01J 37/03, B01J 35/00, C07C 5/48, C07C 51/25

(54) **SYNTHESE EINES MOVTENB KATALYSATORS AUS PREISGUENSTIGEN METALLOXIDEN**
SYNTHESIS OF A MOVTENB CATALYST FROM LOW COST METAL OXIDES
SYSTHÈSE D'UN CATALYSEUR MOVTENB À PARTIR D'OXYDES MÉTALLIQUES PEU COÛTEUX

(30) Priorität: 31.01.2017 DE 102017000861
(43) Veröffentlichungstag der Anmeldung: 11.12.2019
(73) Patentinhaber: Clariant Produkte (Deutschland) GmbH, 65929 Frankfurt am Main (DE)
(72) Erfinder: MESTL, Gerhard, 80935 Muenchen (DE); WANNINGER, Klaus, 83059 Kolbermoor (DE); MELZER, Daniel, 80339 Muenchen (DE); SANCHEZ-SANCHEZ, Maria Cruz, 80469 München (DE); TSEGLAKOVA, Julia, 45476 Muehlheim an der Ruhr (DE); LERCHER, Johannes, 85521 Ottobrunn (DE)
(74) Vertreter: Clariant Produkte (Deutschland) GmbH
(86) Internationale Anmeldenummer: PCT/EP2018/052013
(87) Internationale Veröffentlichungsnummer: WO 2018/141654

(56) Entgegenhaltungen:
- EP-A2- 1 598 112
- DE-A1- 10 119 933
- DE-A1- 102011 109 774
- US-A1- 2014 336 411

## Beschreibung

Die Erfindung betrifft ein neues Verfahren zur Herstellung eines Mischoxidmaterials, das Molybdän, Vanadium, Tellur und Niob enthält als Katalysator für die oxidative Dehydrierung von Ethan zu Ethen oder die Oxidation von Propan zu Acrylsäure und ein Verfahren zur Herstellung des Mischoxidmaterials.

MoVNbTe-Mischoxide zur Oxidation von Propan zu Acrylsäure oder zur oxidativen Dehydrierung von Ethan zu Ethen sind Stand der Technik. Mehr als 200 Patente und zahlreiche wissenschaftliche Veröffentlichungen behandeln Katalysatoren auf Basis von MoVNbTe-Mischoxiden. Die Promotierung dieser Mischoxide mit anderen Metallen des Periodensystems ist bekannt. Dabei liegen die höchsten vorbeschriebenen Acrylsäure-Ausbeuten bei 60% und die von Ethen bei ca. 80%.

Das auf vier Elementen beruhende MoVNbTe-Basissystem für einen Katalysator wurde von Mitsubishi für die Ammoxidation von Propan zu Acrylnitril (1989, EP 318295 A1) und die Oxidation zu Acrylsäure vorgeschlagen (1994, EP 6088038 A2). In JP H07-053414 (Mitsubishi) wird ein katalytisches Verfahren zur Herstellung von Ethylen durch die oxidative Hydrogenierung von Ethan bei tiefer Temperatur, mit einer hohen Ausbeute und mit einer hohen Selektivität, offenbart. Dieses Verfahren zur Herstellung von Ethylen durch das in Kontakt bringen von Ethan mit einem molekularen Sauerstoff enthaltenden Gas in der Anwesenheit einer Katalysatorzusammensetzung bei erhöhter Temperatur, umfasst, dass die Katalysatorzusammensetzung ein Mischmetalloxid enthält, welches als wesentliche Komponenten Molybdän, Vanadium, Tellur und Sauerstoff aufweist und welches ein Pulver-Röntgendiffraktogramm zeigt, das im Wesentlichen die folgenden relativen Peak-Intensitäten aufweist: 2θ (+-0.4°), rel. Int.: 22.1° (100), 28,2° (400~3), 36,2° (80~3), 45.1° (40~3), 50° (50~3).

MoVNbTe-Katalysatoren bestehen hauptsächlich aus zwei orthorhombischen Phasen, die "M1" und "M2" genannt werden (T. Ushikubo, K. Oshima, A. Kayou, M. Hatano, Studies in Surface Science and Catalysis 112, (1997), 473). Die M1-Phase scheint bei den selektiven Oxidationsreaktionen die wesentliche Rolle zu spielen.

Gemäß P. De Santo et al., Z. Kristallogr. 219 (2004) 152 können die Hauptphasen M1 und M2 in Multimetalloxid-Katalysatoren für die selektive Oxidation beispielsweise mit folgenden Summenformeln beschrieben werden:

M1: Mo₁V_{0,15}Te_{0,12}Nb_{0,128}O_{3,7} oder Mo_{7,8}V_{1,2}Te_{0,937}Nb₁O_{28,9}

M2: *Mo₁V_{0,32}Te_{0,42}Nb_{0,08}O_{4,6} oder Mo_{4,31}V_{1,36}Te_{1,81}Nb_{0,33}O_{19,81}

Die beiden Hauptphasen können auch mit etwas anderer Stöchiometrie auftreten. So sind sowohl Vanadium als auch Molybdän im Zentrum eines Oktaeders aus Sauerstoffatomen und daher in der Struktur teilweise austauschbar, so dass die gleiche Struktur z.B. die M1-Phase, auch mit einem höheren Vanadiumgehalt möglich ist. Eine detaillierte Untersuchung dieser Zusammenhänge findet sich bei P. Botella et al., Solid State Science 7 (2005) 507-519. Speziell die M2-Phase ist für die oxidative Dehydrierung von Ethan nicht aktiv (Siehe J.S. Valente et al., ACS Catal. 4(2014), 1292-1301 speziell S.1293). Für die oxidative Dehydrierung von Ethan ist also ein Katalysator, der aus einer möglichst reinen M1-Phase besteht, erwünscht. Es wird daher versucht, diese Kristallphasen sauber und getrennt herzustellen.

EP 529853 A2 offenbart einen Katalysator, der zur Herstellung eines Nitrils aus einem Alkan geeignet ist, wobei der Katalysator die empirische Formel MoV_{b}Te_{c}XₓOₙ hat, worin X mindestens eines von Nb, Ta, W, Ti, Al, Zr, Cr, Mn, Fe, Ru, Co, Rh, Ni, Pd, Pt, Sb, Bi, B und Ce ist, b 0,01 bis 1,0 ist, c 0,01 bis 1,0 ist; x 0,01 bis 1,0 ist und n eine Zahl ist, gemäß der die Gesamtwertigkeit der Metallelemente erfüllt ist und der Katalysator Röntgenstrahlbeugungspeaks bei den folgenden 2θ -Winkeln in seinem Röntgenbeugungsmuster aufweist: Beugungswinkel bei 2θ (22,1° +/- 0,3°, 28,2° +/-0,3°, 36,2° +/- 0,3°, 45,2° +/- 0,3°, 50,0° +/- 0,3°).

JP H07-232071 offenbart ein katalytisches Verfahren zur Herstellung eines Nitrils, bei einer relativ niedrigen Temperatur und mit einer hohen Ausbeute, unter Verwendung eines Alkans als Rohmaterial und eines bestimmten Katalysators. Die Hauptkomponente des Katalysators ist ein Mischmetalloxid aus Molybdän, Vanadium, Tellur, Sauerstoff und X (X ist eines oder mehrere Elemente ausgewählt aus der Gruppe aus Niob, Tantal etc.), wobei das Verhältnis der Hauptkomponenten, d.h. ausgenommen Sauerstoff, ausgedrückt wird, durch die Formeln I bis IV: I) 0,25 < rMo < 0,98, II) 0,003 < rV < 0,50, III) 0,003 < rTe < 0,50, IV) 0 ≤ rX < 0,5, (rMo, rV, rTe und rX sind jeweils die molaren Teile an Molybdän, Vanadium, Tellur und X) und sich im XRD, XRD-Banden dieses Mischoxides bei den verschiedenen 2θ-Winkeln 9.0°±0.3°, 22.1°±0.3°, 27.3°±0.3°, 29.2°±0.3° und 35.4°±0.3° zeigen. Demnach kann ein Nitril hergestellt werden, indem ein Alkan ohne die Anwesenheit einer halogenierten Substanz, z.B. mit Wasser etc., im Reaktionssystem, bei einer tiefen Temperatur mit einer hohen Ausbeute umgesetzt wird.

Andere erfolgreiche Versuche eine reine M1-Phase herzustellen, basieren darauf, die M2-Phase aus dem Phasengemisch herauszulösen. Diese Versuche sind z.B. in EP 1301457 A2, EP 1558569 A1 oder WO 2009106474 A2 beschrieben.

A.C. Sanfiz et al., Top. Catal. 50 (2008) 19-32, beschreiben Hydrothermalsynthesen von MoVNbTe-Oxid. In diesen Synthesen wird ausschließlich von löslichen Verbindungen ausgegangen. Als lösliche Verbindung des Tellurs wird in der Regel Tellursäure Te(OH)₆ eingesetzt. In der gängigsten oxidischen Tellurverbindung TeO₂ hat Tellur die Oxidationsstufe +4. Leider ist Tellurdioxid (TeO₂) schlecht wasserlöslich. In der Tellursäure aber hat das Tellur die Oxidationsstufe +6. Tellur muss also bei der Herstellung der Tellursäure hoch oxidiert werden. Die gängige Synthese erfolgt durch Oxidation von Telluroxid mit Wasserstoffperoxid, was im großen Maßstab Sicherheitsprobleme mit sich bringt, denn Wasserstoffperoxid kann in Selbstzersetzung zu Wasser und Sauerstoff disproportionieren. Deshalb ist Tellursäure nur schwer in großen Mengen herzustellen. US 2014/336411 A1 1 offenbart ein Verfahren zur Herstellung eines Mischoxidmaterials enthaltend die Elemente Molybdän, Vanadium, Niob und Tellur. Die Synthese erfolgte unter hydrothermalen Bedingungen unter Verwendung von Molybdäntrioxid, Vanadiumpentoxid, Nioboxalat und Te(OH)₆ als Ausgangsverbindungen, und in Gegenwart von Oxoliganden wie Ethylenglykol und Zitronsäure.

Eine Synthese des MoVNbTe-Mischoxids ohne Einsatz von Tellursäure hat daher das Potential deutlich kostengünstiger zu sein.

Die in der Synthese von MoVNbTe-Mischoxiden eingesetzte Nb-Komponente ist in der Regel Ammoniumnioboxalat. Nioboxid dagegen ist schwerlöslich und eignet sich daher nur bedingt als Ausgangsverbindung.

Watanabe (Applied Catal. A General, 194-195 (2000) 479-485) beschreibt unter anderem die hydrothermale Synthese aus den wenig löslichen Vorstufen MoO₃, V₂O₅ und TeO₂. Die Hydrothermalsynthese ergibt eine Vorstufe für einen Ammoxidations-Katalysator, der im Vergleich zu einem Katalysator, der durch die bekannte trockene Methode hergestellt wird, die doppelt so hohe Aktivität nach der Kalzinierung aufweist. Die Mischoxide, die durch die Feststoffreaktion hergestellt werden, zeigen eine eher geringe Aktivität. Es wurde vorgeschlagen, dass die höhere Aktivität des durch die Hydrothermalsynthese hergestellten Katalysators vor allem mit der höheren Oberfläche zu tun hat.

Die Aufgabe der vorliegenden Erfindung ist es, ein Mischoxidmaterial enthaltend Molybdän, Vanadium, Tellur und Niob ("MoVTeNb-Mischoxid"), mit einem hohen Gehalt an M1-Phase bereitzustellen, das als Katalysatormaterial speziell für die Oxidation von Ethan zu Ethylen eine möglichst hohe Aktivität und Selektivität aufweist.

Diese Aufgabe wird gelöst, durch ein Verfahren zur Herstellung eines Mischoxidmaterials, umfassend die Schritte:
a) Herstellen eines Gemisches aus Ausgangsverbindungen, das Molybdän, Vanadium, Niob und Tellur enthaltende Ausgangsverbindungen sowie zwei chelatisierende Oxoliganden enthält,
b) hydrothermale Behandlung des Gemisches aus Ausgangsverbindungen bei einer Temperatur von 100 °C bis 300 °C, um eine Produktsuspension zu erhalten,
c) Abtrennen und Trocknen des Feststoffes, der in der aus Schritt b) resultierenden Produktsuspension enthalten ist,
d) Aktivieren des aus Schritt c) erhaltenen Feststoffes in inertem Gas.

Das Gemisch aus Ausgangsverbindungen liegt vorzugsweise als wässrige Suspension vor und wird anschließend hydrothermal behandelt. Der Begriff "hydrothermal" bezieht sich vorwiegend auf Reaktionsbedingungen zur Herstellung eines Katalysatormaterials in Gegenwart von Wasser und unter erhöhter Temperatur und/oder erhöhtem Druck, beispielsweise im Autoklaven. Dabei kann der Druck im Bereich von 5 bis 30 bar, bevorzugt von 10 bis 27 bar liegen. Beispielhafte Druckbereiche sind 11 bis 20 bar.

Durch die hydrothermale Behandlung (Schritt b)) wird eine Produktsuspension erhalten, die das MoVNbTe-Mischoxid als Feststoff enthält. In dem erfindungsgemäßen Verfahren kann das Abtrennen des Feststoffs der Suspension in Schritt c) durch einen oder mehrere Schritte der Filtration, z.B. des Abfiltrierens von der Mutterlauge, erfolgen. Das Trocknen kann in einem Schritt durchgeführt werden oder in zwei Schritten in strömender oder statischer Luft. Dabei ist der erste Trocknungsschritt bevorzugt bei 60 °C bis 150 °C (besonders bevorzugt bei 80 °C bis 120°C) und der zweite Trocknungsschritt bei 200 °C bis 350 °C (besonders bevorzugt bei 220 °C bis 280 °C) durchzuführen. Zusätzlich kann Schritt c) des erfindungsgemäßen Verfahrens einen oder mehrere Schritte des Waschens, des Kalzinierens (thermische Behandlung), und/oder des Mahlens beinhalten. Das Kalzinieren kann bei 200 bis 500 °C bevorzugt 250 °C bis 350 °C an Luft erfolgen.

Nach dem Trocknen des Filtrates in Schritt c) wird die getrocknete Mischung z.B. in einer strömenden oder statischen Inertgasatmosphäre bei ungefähr 500 °C bis 700 °C für mindestens 1 Stunde aktiviert (Schritt d). Als Inertgas eignet sich insbesondere Stickstoff, Helium oder Argon. Bevorzugt ist es, wenn das Aktivieren im Bereich von 550 °C bis 650 °C erfolgt. Z.B. kann das Aktivieren bei ungefähr 600 °C für ungefähr 2 Stunden erfolgen.

Die Ausgangsverbindungen sind Molybdäntrioxid, Vanadiumpentoxid, Niobpentoxid und eine Tellur enthaltende Ausgangsverbindung, wobei die Tellur enthaltende Ausgangsverbindung Tellurdioxid ist.

Tellurdioxid kann bevorzugt in einem beliebigen Hydratisierungs-Grad vorliegen.

Bevorzugt weist Tellurdioxid eine Partikelgröße mit einem D₉₀ < 100 µm auf.

Besonders bevorzugt gilt, dass der D₅₀-Wert für das als Ausgangsverbindung eingesetzte Tellurdioxid unter 35 µm liegt.

Nioboxid kann ebenfalls eine Partikelgröße mit einem D₉₀ < 100 µm, bevorzugt D₉₀ < 75 µm, besonders bevorzugt D₉₀ < 50 µm aufweisen. Optional kann Nioboxid eine Partikelgröße D₅₀ < 50 µm oder < 35 µm aufweisen.

Außerdem können alle eingesetzten Ausgangsverbindungen eine Partikelgröße mit einem D₉₀ < 100 µm, bevorzugt D₉₀ < 75 µm, besonders bevorzugt D₉₀ < 50 µm aufweisen. Optional können die Ausgangsverbindungen eine Partikelgröße D₅₀ < 50 µm oder < 35 µm aufweisen.

Die Ausgangsverbindungen liegen als Pulver vor und weisen eine Partikelgrößenverteilung auf. Die Partikelgröße D₉₀ ist definiert als die Grenze des Partikeldurchmessers in der Partikelgrößenverteilung, unter der 90% aller Partikel liegen. Die Partikelgröße des Meridians, also die Partikelgröße unter der die Hälfte aller Partikel in der Partikelgrößenverteilung liegen, wird auch als Partikelgröße D₅₀ bezeichnet. Als besonders bevorzugt gilt, dass die Partikelgröße D₅₀ für das als Ausgangsverbindung eingesetzte Tellurdioxid unter 35 µm liegt.

Die gewünschte Partikelgröße D₉₀ oder D₅₀ der Ausgangsverbindung kann erhalten werden, indem von einem Pulver mit einer grobkörnigeren Partikelgrößenverteilung ausgegangen wird und die Partikel mechanisch zerkleinert werden. Dies kann durch Vermahlen erfolgen, wobei alle geeigneten und dem Fachmann geläufigen Mittel Verwendung finden können, so z.B. Schlagmühlen, Planetenmühlen, Mörser etc.

Ein Vorteil des erfindungsgemäßen Herstellungsverfahrens ist es, dass eine Synthese der M1-Phase aus den unlöslichen und preisgünstigen Oxiden, MoO₃, V₂O₅, Nb₂O₅ und TeO₂ als Ausgangsverbindungen verwendet werden. Als chelatisierende Oxoliganden haben sich besonders Zitronensäure, Glykol und Oxalsäure als geeignet herausgestellt.

Die beiden chelatisierenden Oxoliganden sollte vorzugsweise im Gemisch der Ausgangsverbindungen jeweils in einem Mo/Oxoliganden-Verhältnis von 1:0,01 bis 1:1, bevorzugt 1:0,08 bis 1:0,4, stärker bevorzugt 1:0,15 bis 1:0,25 vorliegen. Die beiden chelatisierenden Oxoliganden können hierbei jeweils dasselbe Mo/Oxoliganden-Verhältnis aufweisen oder ein unterschiedliches.

Die erfindungsgemäße Synthese liefert nicht nur ein MoVTeNb-Mischoxid mit einer hohen Phasenreinheit in Bezug auf die M1-Phase, sondern das erhaltene MoVTeNb-Mischoxid stellt auch ein Katalysatormaterial mit verbesserter Aktivität für die Oxidation von Ethan zu Ethylen dar. Ein weiterer Vorteil der erfindungsgemäßen Synthese der M1-Phase ist die hohe Effizienz der Umsetzung der Ausgangsstoffe durch die Hydrothermalsynthese.

Sofern sich die Stöchiometrie der Edukte im Bereich Mo/V/Nb/Te = 1:0,22:0,1:0,1 bis 1:0,3:0,17:0,17 bewegt, werden Mo, V, Nb und Te fast vollständig zur M1-Phase umgesetzt, sodass weniger als 100 ppm aller Metalle in der Mutterlauge verbleiben. Die mögliche Stöchiometrie der M1-Phase ist aus der Literatur hinreichend bekannt und lässt sich durch die Formel Mo₁VₐNb_{b}Te_{c}Oₓ mit a = 0,2 bis 0,3, b = 0,1 bis 0,2, c = 0,1 bis 0,25 und x abhängig von der Oxidationsstufe der Metalle (Mo, V, Nb und Te) eine Größe die zum Ladungsausgleich führt.

Es wurde außerdem gefunden, dass bei Verwendung von MoO₃, V₂O₅, Nb₂O₅ und TeO₂ mit Zitronensäure, Glykol und Oxalsäure die hydrothermale Kristallisation hervorragend gelingt. Besonders gut gelingt die Kristallisation, in der Abwesenheit von Ammoniumionen. Bevorzugt ist daher, die Abwesenheit von Ammoniumionen bei der Synthese.

Durch das erfindungsgemäße Verfahren wird ein Mischoxidmaterial umfassend die Elemente Molybdän, Vanadium, Niob und Tellur, für die Oxidation von Ethan, erhalten, mit folgender Stöchiometrie:

Mo₁VₐNb_{b}Te_{c}Oₓ

mit 0,27 < a < 0,31; 0,08 < b < 0,12; 0,08 < c < 0,12,
das im XRD Beugungsreflexe h, i, k und 1 aufweist, deren Scheitelpunkte ungefähr bei den Beugungswinkeln (2·) 26,2° ± 0,5° (h), 27,0° ± 0,5° (i), 7,8° ± 0,5° (k) und 28,0° ± 0,5° (1) liegen.

Das erfindungsgemäße MoVTeNb-Mischoxid kann als Katalysatormaterial zur Oxidation und/oder oxidativen Dehydrierung ("ODH") von Kohlenwasserstoffen, insbesondere zur oxidativen Dehydrierung von Ethan zu Ethylen verwendet werden. Es kann eine BET-Oberfläche von 5 bis 25 m²/g, besonders bevorzugt von 5 bis 15 m²/g aufweisen.

Das MoVTeNb-Mischoxid, das nach dem erfindungsgemäßen Verfahren hergestellt wird, kann als Katalysator, bzw. Katalysatormaterial verwendet werden. Es kann auf verschiedene Weisen in einem kommerziellen Katalysator eingesetzt werden. Z.B. kann es durch Tablettieren zu Katalysatortabletten verarbeitet werden, die dann in einen Reaktor eingefüllt werden können.

Das Katalysatormaterial kann auch zusammen mit einem geeigneten Bindemittel zu einem Extrudat (Tabletten, Formkörper, Honigwabenkörper und dergleichen) verarbeitet werden. Als Bindemittel kann jedes dem Fachmann geläufige und geeignet erscheinende Bindemittel verwendet werden. Bevorzugte Bindemittel sind unter anderem Pseudoböhmit sowie silicatische Bindemittel wie kolloidales Siliciumoxid oder Silicasol.

Das Katalysatormaterial kann ferner zusammen mit anderen Komponenten, vorzugsweise mit einem Bindemittel, besonders bevorzugt mit einem organischen Bindemittel, beispielsweise einem organischen Kleber, Polymeren, Harzen oder Wachsen, zu einem Washcoat verarbeitet werden, der auf einen metallischen oder keramischen Träger aufgebracht werden kann. Gegebenenfalls können zusätzliche Imprägnierschritte oder Kalzinierschritte erfolgen.

Das Röntgendiffraktogramm des nach dem erfindungsgemäßen Verfahren gebildeten MoVTeNb-Mischoxids weist, bei Verwendung der Cu-Kα-Strahlung, Beugungsreflexe h, i, k und l auf, deren Scheitelpunkte ungefähr bei den Beugungswinkeln (2θ) 26,2° ± 0,5° (h), 27,0° ± 0,5° (i), 7,8° ± 0,5° (k) und 28,0° ± 0,5° (1) liegen, wobei die Intensitäten Pₕ, Pᵢ, Pₖ, Pₗ der Beugungsreflexe h, i, k und 1 die folgenden Beziehungen erfüllen können, mit Rₓ (x = 1 bis 3) als das durch die Beziehungen definierte Intensitätsverhältnis: R1 = Ph / (Ph + Pi) > 0,3, bevorzugt > 0,35 und besonders bevorzugt > 0,4;und/oder R2 = Pi / (Pi + Pl) > 0,5, bevorzugt > 0,6 und besonders bevorzugt > 0,63; und/oder R3 = Pi /(Pi + Pk) < 0,8, bevorzugt < 0,75, besonders bevorzugt < 0,7.

Im Röntgendiffraktogramm (XRD) von Ausführungsformen des Mischoxidmaterials kann der Beugungsreflex i die zweithöchste Intensität besitzen und/oder der Beugungsreflex h die dritthöchste Intensität besitzen.

Das erfindungsgemäße MoVNbTe-Mischoxid wird in den Beispielen als Katalysatormaterial eingesetzt und bei den experimentellen Angaben daher teilweise als Katalysator bezeichnet.

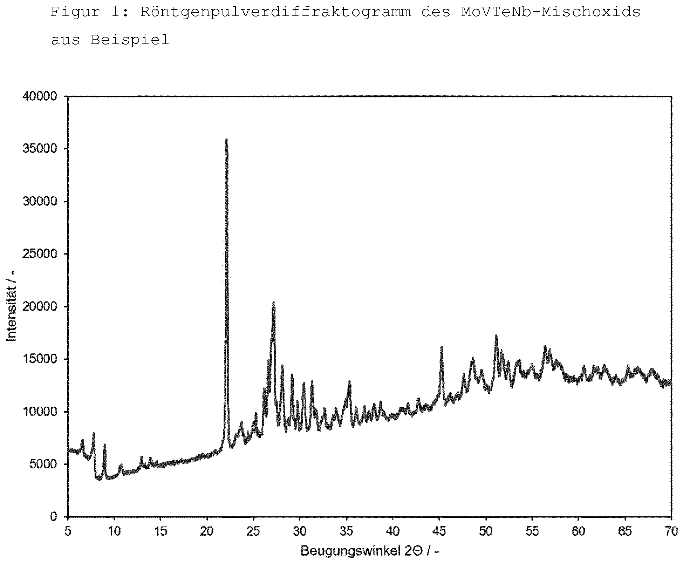

Figur 1: XRD des MoVTeNb-Mischoxids aus Beispiel 1.
Figur 2: XRD des MoVTeNb-Mischoxids aus Beispiel 2.

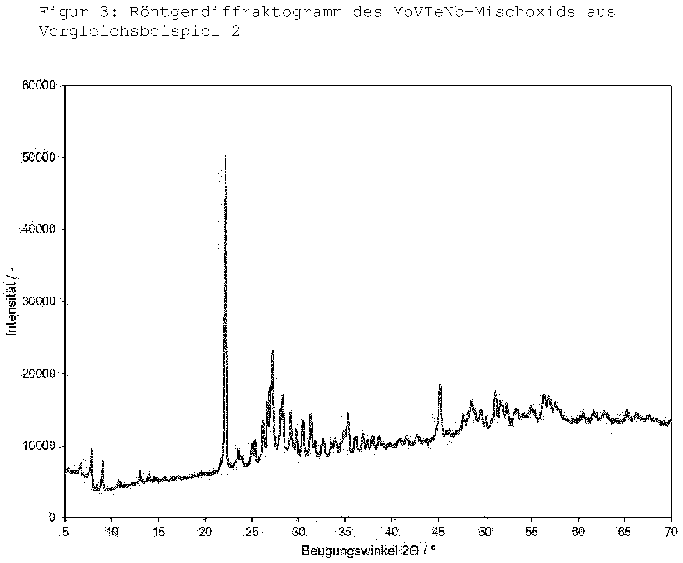

Figur 3: XRD des MoVTeNb-Mischoxids aus Vergleichsbeispiel 1.

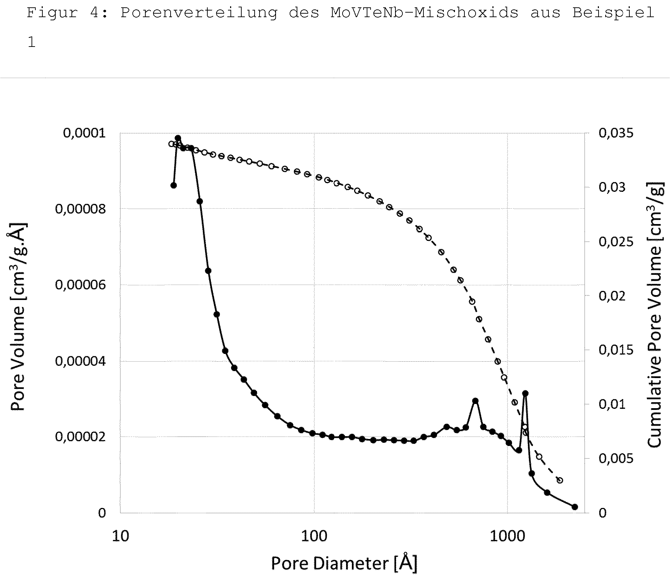

Figur 4: Porenverteilung des MoVTeNb-Mischoxids aus Beispiel 1.

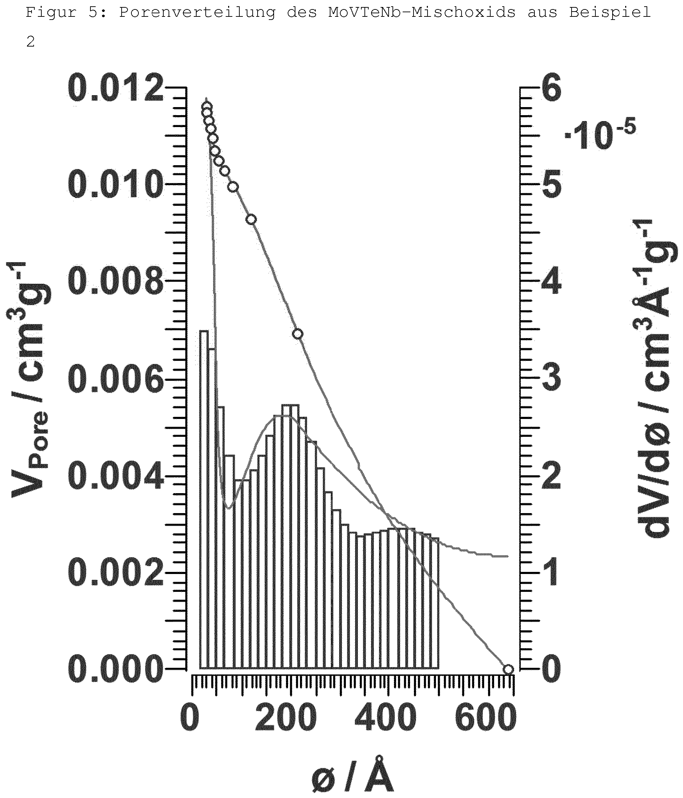

Figur 5: Porenverteilung des MoVTeNb-Mischoxids aus Beispiel 2.

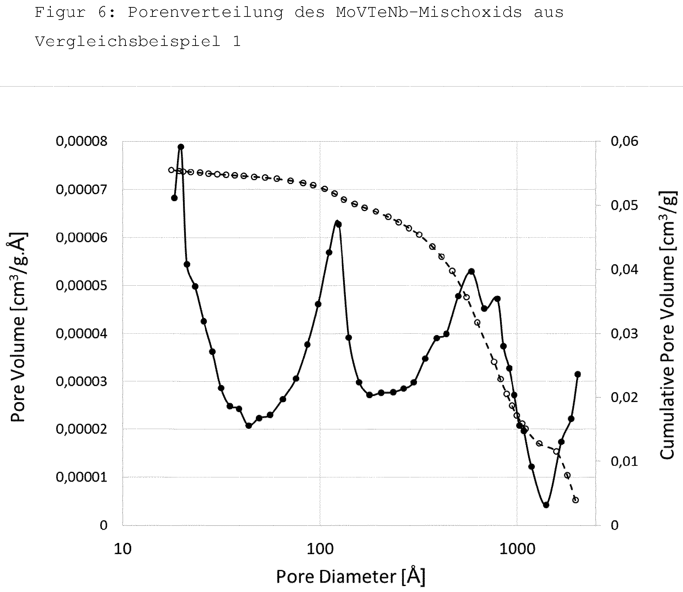

Figur 6: Porenverteilung des MoVTeNb-Mischoxids aus Vergleichsbeispiel 1.
Figur 7: Vergleich der katalytischen Aktivität der MoVTeNb-Mischoxide gemäß Beispiel 2 und Vergleichsbeispiel 1 in der ODH von Ethan.

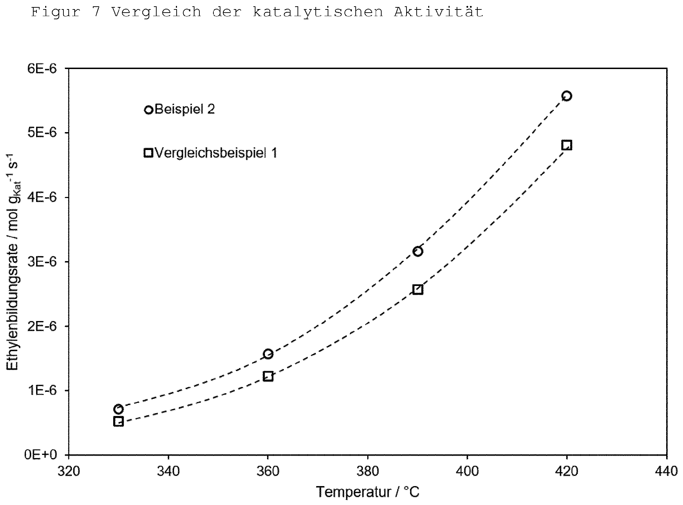

Das nach dem erfindungsgemäßen neuen Verfahren hergestellte MoVTeNb-Mischoxid ist eindeutig aktiver. Es erzielt normiert auf 1 g Katalysator eine höhere Aktivität, als der Katalysator nach dem Stand der Technik gemäß dem Vergleichsbeispiel (Figur 7). Dies belegt, dass mit dem erfindungsgemäßen Verfahren ein Mischoxidmaterial mit neuen Eigenschaften erhalten wird. Jedoch lassen sich die neuen Eigenschaften des neuen Mischoxidmaterials nicht ohne Weiteres mit gängigen Charakterisierungsmethoden erfassen.

### Charakterisierungsmethoden:

Zur Bestimmung der Parameter der erfindungsgemäßen Katalysatoren werden die nachstehenden Methoden eingesetzt:

### 1. BET-Oberfläche

Die Bestimmung erfolgt nach der BET-Methode gemäß DIN 66131; eine Veröffentlichung der BET-Methode findet sich auch in J. Am. Chem. Soc. 60,309 (1938). Die Messungen wurden an einem Sorptomatic 1990 Gerät bei 77 K durchgeführt. Vor der Messung wurde die Probe für 2 h bei 523 K evakuiert. Die lineare Regression der Isothermen nach der BET-Methode wurde in einem Druckbereich von p/p₀ = 0,01 - 0,3 (p₀ = 730 Torr) durchgeführt.

### 2. Pulverröntgendiffraktometrie (XRD)

Die Röntgendiffraktogramme wurde auf einem PANalytical Empyrean, ausgestattet mit einem Medipix PIXcel 3D Detektor, in Θ-Θ Geometrie in einem Winkelbereich von 2Θ = 5 - 70 ° aufgenommen. Die Röntgenröhre erzeugte Cu-K-Strahlung. Die Cu-Kβ-Strahlung wurde durch Verwendung eines Ni-Filters im Strahlengang es einfallenden Röntgenstrahls unterdrückt, so dass an der Probe ausschließlich Cu-Kα-Strahlung mit einer Wellenlänge von 15,4 nm (E = 8,04778 keV) gebeugt wurde. Die Höhe des quellenseitigen Strahlenganges wurde mittels automatischem Divergenzschlitz (programmable diveregence slit - PDS) so angepasst, dass die Probe über den gesamten Winkelbereich auf einer Länge von 12 mm bestrahlt wurde. Die Breite des detektorseitigen Röntgenstrahls wurde durch eine feste Blende auf 10 mm begrenzt. Horizontale Divergenz wurde durch Verwendung eines 0.4 rad Soller Slits minimiert.

Die Höhe des detektorseitigen Strahlenganges wurde analog zum quellenseitigen Strahlengang mittels automatischem Antibrechungsschlitz (programmable anti-scatter slit - PASS) so angepasst, dass über den gesamten Winkelbereich der auf einer Länge von 12 mm auf der Probe reflektierten Röntgenstrahl detektiert wurde.

Die Proben wurden, je nach vorhandener Menge, entweder auf einem amorphen Siliziumprobenteller oder tablettiert als Flachbettproben präpariert.

### 3. Porenverteilung

Die Porengrößenverteilung wurde mittels Stickstoffsorptionsmessungen an einem Sorptomatic Gerät oder einem TriStar 3000 Gerät bei 77 K durchgeführt. Vor der Messung wurde die Probe für 2 h bei 523 K evakuiert. Es wurden sowohl Ad- als auch Desorptionsisothermen bestimmt und zur Auswertung nach der Barrett-Joyner-Halenda Methode (BJH) herangezogen.

Die Erfindung soll nun anhand der nachstehenden und nicht als einschränkend zu verstehenden Ausführungsbeispiele näher erläutert werden.

### Ausführungsbeispiele:

### Beispiel 1:

TeO₂ (Alfa Aesar) wurde in 200 g dest. H₂O aufgeschlämmt und in einer Planetenkugelmühle mit 1 cm Kugeln (ZrO₂) gemahlen. Anschließend wurde die Portion mit 500 ml dest. H₂O in ein Becherglas überführt. Das Nb₂O₅ wurde in 200 g dest. H₂O aufgeschlämmt und in der gleichen Kugelmühle gemahlen. Anschließend wurde die Portion mit 500 ml dest. H₂O in ein Becherglas überführt. Am nächsten Morgen wurde auf 80 °C aufgeheizt, 107,8 g Oxalsäure-Dihydrat in die Nb₂O₅ Suspension gegeben und für ca. 1 h gerührt. Im Autoklaven (40 L) wurden 6 L dest. H₂O vorgelegt und unter Rühren (Geschwindigkeit des Rührers 90 rpm) auf 80 °C erhitzt. Hat das Wasser die Temperatur erreicht wurde nacheinander 61,58 g Zitronensäure, 19,9 g Ethylenglykol, 615,5 g MoO₃ (Sigma Aldrich), 124,5 g V₂O₅, das gemahlene TeO₂ und das gemahlene Nb₂O₅ in Oxalsäure zugegeben. 850 ml dest. H₂O wurden zum Überführen und Spülen der Gefäße verwendet. Die komplette Wassermenge im Autoklav beträgt 8,25 L. Anschließend wurde mit Stickstoff überlagert. Es wurde eine Hydrothermalsynthese im 40 L Autoklaven bei 190 °C/48 h durchgeführt. Nach der Synthese wurde mit Hilfe einer Vakuumpumpe mit Blaubandfilter abfiltriert und der Filterkuchen mit 5 L dest. H₂O gewaschen.

Die Trocknung erfolgte bei 80 °C im Trockenschrank für 3 Tage und anschließend wurde das Produkt in einer Schlagmühle gemahlen. Es wurde eine Feststoff-Ausbeute von 0,8 kg erreicht.

Die anschließende Kalzinierung erfolgte bei 280 °C für 4 h an Luft (Heizrate 5 °C/min Luft: 1 L/min).

Die Aktivierung erfolgte in einer Retorte bei 600 °C für 2 h (Heizrate 5 °C/min N₂: 0,5 L/min).

Das Produkt wies eine BET-Oberfläche von 9 m²/g und ein Porenvolumen = 0,04 cm³/g auf.

### Beispiel 2:

In einem 100 mL PTFE-Becher wurde 75 mL bidestilliertes Wasser vorgelegt, 177,8 mg (Mono-)Ethylenglykol zugetropft und anschließend wurde 5397,9 mg MoO₃, 1023,9 mg V₂O₅, 599,1 mg TeO₂, 549,5 mg Nb₂O₅·xH₂O (Nb = 63,45 Gew.-%), 540,9 mg Zitronensäure und 338,3 mg Oxalsäure aufgeschlämmt. Der Teflonbecher wird verschlossen und in eine Edelstahlautoklavenbombe überführt. Diese wurde druckdicht verschlossen und in einem auf 190 °C vorgeheizt Ofen auf eine horizontal drehende Welle gespannt. Nach 48 Stunden wurde die Autoklavenbombe aus dem Ofen entnommen und sofort unter fließendem Wasser abgeschreckt und anschließend in einem Eisbad für 45 Minuten abgekühlt.

Die entstandene Produktsuspension wurde über Filterpapier (Porenweite 3 µm) abfiltriert und der Feststoff mit 200 mL bidestilliertem Wasser gewaschen.

Das so gewonnene Produkt wurde für 16 h in einem Trockenschrank bei 80 °C getrocknet und danach in einem Handmörser zermahlen.

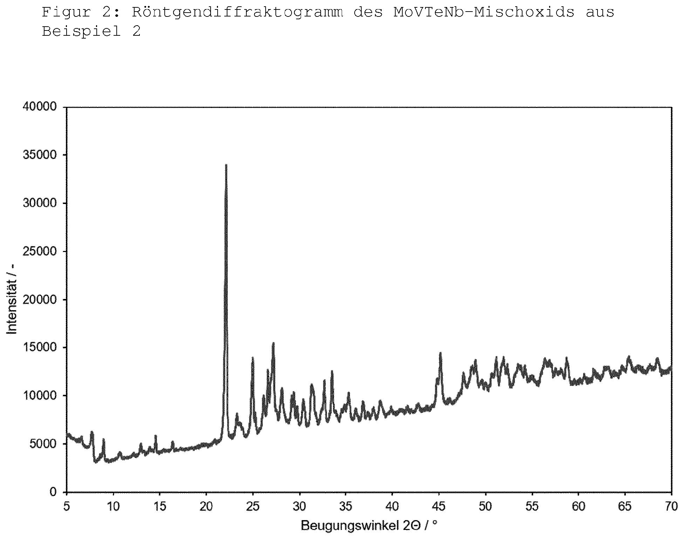

Die Feststoff-Ausbeute betrug 6,2 g. Die Aktivierung erfolgte bei 600 °C für 2 h (Heizrate 10 °C/min N₂: 100 mL/min). Das XRD-Diffraktogramm des Produkts wird in Figur 2 gezeigt, die BET-Oberfläche betrug 7,3 m²/g, das Porenvolumenlag lag unter 0,012 cm³/g.

### Vergleichsbeispiel 1:

Im Autoklaven (40 L) werden 3,3 L dest. H₂O vorgelegt und unter Rühren auf 80°C erhitzt. Währenddessen wird 725,58 g Ammoniumheptamolybdat-Tetrahydrat (von HC Starck) hineingegeben und gelöst (AHM-Lösung). In drei 5 L Bechergläsern werden jeweils 1,65 L dest. H₂O unter Rühren auf einem Magnetrührer mit Temperaturregelung ebenfalls auf 80 °C erhitzt. In diese Bechergläser wurden dann jeweils 405,10 g Vanadylsulfathydrat (GfE, V Gehalt: 21,2%), 185,59 g Ammoniumnioboxalat (HC Starck, Nb Gehalt: 20,6%) und 94,14 g Tellursäure zugegeben und gelöst (V-Lösung, Nb-Lösung und Te-Lösung).

Anschließend wurden nacheinander die V-Lösung, die Te-Lösung und zum Schluss die Nb-Lösung in die AHM-Lösung mittels einer Schlauchpumpe gepumpt. Pumpzeit: V-Lösung: 4,5 min mit 190 rpm (Schlauchdurchmesser: 8x5 mm), Nb-Lösung: 6 min mit 130 rpm (Schlauchdurchmesser: 8x5 mm).

Die entstandene Suspension wurde nun 10 min bei 80 °C weitergerührt. Die Geschwindigkeit des Rührers bei der Fällung betrug 90 rpm.

Anschließend wurde mit Stickstoff überlagert, indem im Autoklaven mit Stickstoff ein Druck bis ca. 6 bar aufgebaut und das Ablassventil so weit geöffnet wurde, dass der Autoklav unter Druck von N₂ durchströmt wurde (5 min). Am Ende wurde der Druck, über das Entlüftungsventil, bis auf 1 bar Restdruck wieder abgelassen.

Die Hydrothermalsynthese im 40 L Autoklaven wurde bei 175 °C für 20 h (Aufheizzeit: 3 h), mit einem Ankerrührer bei einer Rührergeschwindigkeit von 90 rpm durchgeführt.

Nach der Synthese wurde mit Hilfe einer Vakuumpumpe mit Blaubandfilter abfiltriert und der Filterkuchen mit 5 L dest. H₂O gewaschen.

Die Trocknung erfolgte bei 80 °C im Trockenschrank für 3 Tage und anschließend wurde in einer Schlagmühle gemahlen, die Feststoff-Ausbeute betrug 0,8 kg.

Die Kalzinierung erfolgte bei 280 °C für 4 h (Heizrate von 5 °C/min Luft: 1 L/min). Die Aktivierung erfolgte in der Retorte bei 600 °C für 2 h (Heizrate 5°C/min N₂: 0,5 L/min).

Die BET-Oberfläche des Produkts betrug 9 m²/g, das Porenvolumen = 0.055 cm³/g.

### Beispiel 3:

Die katalytische Aktivität in der oxidativen Dehydrierung von Ethan der Katalysatoren aus Beispiel 2 und dem Vergleichsbeispiel wurde in einem Rohrreaktor bei Atmosphärendruck im Temperaturbereich 330 bis 420 °C untersucht. Dazu wurden 25 mg (Beispiel 2) oder 200 mg (Vergleichsbeispiel 1) Katalysator (Korngröße 150 bis 212 µm) mit Silizumcarbid (Korngröße 150 bis 212 µm) im Massenverhältnis 1 : 5 verdünnt. Unter- und oberhalb des Katalysatorbettes wurde eine Schicht aus jeweils 250 mg Siliziumcarbid der gleichen Korngröße eingefüllt und die Enden des Rohrreaktors durch Quarzwollepfropfen verschlossen.

Der Reaktor wurde vor Beginn des Experiments mit Inertgas gespült und anschließend unter einem Heliumfluss von 50 sccm auf 330 °C aufgeheizt. Nachdem die gewünschte Temperatur erreicht wurde und für eine Stunde stabil war, wurde auf das Reaktionsgasgemisch umgeschalten.

Die Eingangsgaszusammensetzung war C₂H₆/O₂/He = 9,1/9,1/81,8 (v/v) bei einem Gesamtvolumenstrom von 50 sccm.

Die Analyse des Produktgasstromes wurde in einem Gaschromatographen ausgerüstet mit Haysep N- und Haysep Q-Säulen, einer Molsiebsäule 5A und einem Wärmeleitfähigkeitsdetektor bestimmt.

Die Ethylenbildungsraten unter den oben beschriebenen Bedingungen sind in Figur 7 dargestellt.

## Patentansprüche

1. Verfahren zur Herstellung eines Mischoxidmaterials, umfassend die Schritte:
a) Herstellen eines Gemisches aus Ausgangsverbindungen, das Molybdäntrioxid, Vanadiumpentoxid, Niobpentoxid und eine Tellur enthaltende Ausgangsverbindung sowie zwei chelatisierende Oxoliganden enthält,
b) hydrothermale Behandlung des Gemisches aus Ausgangsverbindungen bei einer Temperatur von 100 °C bis 300 °C, um eine Produktsuspension zu erhalten,
c) Abtrennen und Trocknen des Feststoffes, der in der aus Schritt b) resultierenden Produktsuspension enthalten ist,
d) Aktivieren des aus Schritt c) erhaltenen Feststoffes in inertem Gas, **dadurch gekennzeichnet, dass** die Tellur enthaltende Ausgangsverbindung Tellurdioxid ist.

2. Verfahren nach Anspruch 1, **dadurch gekennzeichnet, dass** das Aktivieren in Schritt d) bei einer Temperatur zwischen 450 °C bis 700 °C, bevorzugt 550 °C bis 650 °C erfolgt.

3. Verfahren nach Anspruch 2, **dadurch gekennzeichnet, dass** das Niobpentoxid eine Partikelgröße D₉₀ kleiner 100 µm aufweist.

4. Verfahren nach Anspruch 1, **dadurch gekennzeichnet, dass** das Tellurdioxid eine Partikelgröße D₉₀ kleiner 100 µm aufweist.

5. Verfahren nach einem der vorhergehenden Ansprüche, **dadurch gekennzeichnet, dass** einer der chelatisierenden Oxoliganden Ethylenglykol ist.

6. Verfahren nach einem der vorhergehenden Ansprüche, **dadurch gekennzeichnet, dass** einer der chelatisierenden Oxoliganden Zitronensäure ist.

7. Ein Mischoxidmaterial für die Oxidation von Ethan, umfassend die Elemente Molybdän, Vanadium, Niob und Tellur, mit folgender Stöchiometrie:
Mo₁VₐNb_{b}Te_{c}Oₓ
mit 0,27 < a < 0,31; 0,08 < b < 0,12; 0,08 < c < 0,12,
das im XRD, bei Verwendung der Cu-Kα-Strahlung,
Beugungsreflexe h, i, k und 1 aufweist, deren Scheitelpunkte ungefähr bei den Beugungswinkeln (2θ) 26,2° ± 0,5° (h), 27,0° ± 0,5° (i), 7,8° ± 0,5° (k) und 28,0° ± 0,5° (1) liegen, herstellbar nach dem Verfahren aus Anspruch 1.

8. Verwendung eines Katalysators nach Anspruch 7 zur oxidativen Dehydrierung von Ethan zu Ethen.

9. Verwendung eines Katalysators nach Anspruch 7 zur Oxidation von Propan.

## Claims

1. A process for preparing a mixed oxide material comprising the steps of:
a) Preparation of a mixture of starting compounds containing molybdenum trioxide, vanadium pentoxide, niobium pentoxide and a starting compound containing tellurium and two chelating oxoligands,
b) hydrothermal treatment of the mixture of starting compounds at a temperature of 100 °C to 300 °C to obtain a product suspension,
c) separating and drying the solid contained in the product suspension resulting from step b),
d) activating the solid obtained from step c) in inert gas, **characterized in that** the tellurium-containing starting compound is tellurium dioxide.

2. Process according to claim 1, **characterized in that** the activation in step d) is carried out at a temperature between 450 °C to 700 °C, preferably 550 °C to 650 °C.

3. Process according to claim 2, **characterized in that** the niobium pentoxide has a particle size D₉₀ of less than 100 µm.

4. Process according to claim 1, **characterized in that** the tellurium dioxide has a particle size D₉₀ of less than 100 µm.

5. Process according to one of the preceding claims, **characterized in that** one of the chelating oxoligands is ethylene glycol.

6. Process according to one of the preceding claims, **characterized in that** one of the chelating oxoligands is citric acid.

7. A mixed oxide material for the oxidation of ethane comprising the elements molybdenum, vanadium, niobium and tellurium, having the following stoichiometry:
Mo₁VₐNb_{b}Te_{c}Oₓ
with 0.27< a < 0.31; 0.08 < b < 0.12; 0.08 < c < 0.12,
which in XRD, using Cu-Kα-radiation, has diffraction reflections h, i, k and l, the apexes of which lie approximately at the diffraction angles (2θ) 26.2° ± 0.5° (h), 27.0° ± 0.5° (i), 7.8° ± 0.5° (k) and 28.0° ± 0.5° (1), obtainable by process of claim 1.

8. Use of a catalyst according to claim 7 for the oxidative dehydrogenation of ethane to ethene.

9. Use of a catalyst according to claim 7 for the oxidation of propane.

## Revendications

1. Procédé de fabrication d'un matériau d'oxyde mixte, comprenant les étapes consistant à:
a) Préparation d'un mélange de composés de départ contenant du trioxyde de molybdène, du pentoxyde de vanadium, du pentoxyde de niobium et un composé de départ contenant du tellure ainsi que deux ligands oxo chélatants,
b) traitement hydrothermique du mélange de composés de départ à une température entre 100 °C à 300 °C, pour obtenir une suspension de produits,
c) séparation et séchage du solide contenue dans la suspension de produit résultant de l'étape b),
d) activer le solide obtenu à l'étape c) dans un gaz inerte, **caractérisé en ce que** le composé de départ contenant du tellure est le dioxyde de tellure.

2. Procédé selon la revendication 1, **caractérisé en ce que** l'activation dans l'étape d) est effectuée à une température entre 450°C à 700°C, de préférence entre 550°C à 650°C.

3. Procédé selon la revendication 2, **caractérisé en ce que** le pentoxyde de niobium présente une taille de particules D₉₀ inférieure à 100 µm.

4. Procédé selon la revendication 1, **caractérisé en ce que** le dioxyde de tellure présente une taille de particules D₉₀ inférieure à 100 µm.

5. Procédé selon l'une des revendications précédentes, **caractérisé en ce qu'**un des oxoligands chélatants est l'éthylèneglycol.

6. Procédé selon l'une des revendications précédentes, **caractérisé en ce que** l'un des oxoligands chélatants est l'acide citrique.

7. Matériau d'oxyde mixte pour l'oxydation de l'éthane, comprenant les éléments molybdène, vanadium, niobium et tellure, ayant la stoechiométrie suivante:
Mo₁VₐNb_{b}Te_{c}Oₓ
avec 0,27 < a < 0,31; 0,08 < b < 0,12; 0,08 < c < 0,12,
qui présente en XRD, en utilisant le rayonnement Cu-Kα, des réflexions de diffraction h, i, k et 1, dont les sommets se situent approximativement aux angles de diffraction (2θ) 26,2° ± 0,5° (h), 27,0° ± 0,5° (i), 7,8 ± 0,5° (k) et 28,0° ± 0,5° (1), pouvant être fabriqué selon le procédé de la revendication 1.

8. Utilisation d'un catalyseur selon la revendication 7 pour la déshydrogénation oxydante de l'éthane en éthène.

9. Utilisation d'un catalyseur selon la revendication 7 pour l'oxydation du propane.
